## Europäisches Patentamt

## European Patent Office

## Office européen des brevets

(19)

(11) Veröffentlichungsnummer : **0 104 187**
**B1**

(12) **EUROPÄISCHE PATENTSCHRIFT**

(45) Veröffentlichungstag der Patentschrift :
**12.02.86**

(51) Int. Cl.⁴ : **A 61 F 2/18**

(21) Anmeldenummer : **83900908.1**

(22) Anmeldetag : **26.03.83**

(86) Internationale Anmeldenummer :
**PCT/DE 83/00057**

(87) Internationale Veröffentlichungsnummer :
**WO/8303350 (13.10.83 Gazette 83/24)**

(54) **GEHÖRKNÖCHELCHEN-PROTHESE UND VERFAHREN ZU IHRER HERSTELLUNG.**

(30) Priorität : **26.03.82 DE 3211209**

(43) Veröffentlichungstag der Anmeldung :
**04.04.84 Patentblatt 84/14**

(45) Bekanntmachung des Hinweises auf die Patenterteilung : **12.02.86 Patentblatt 86/07**

(84) Benannte Vertragsstaaten :
**DE FR GB**

(56) Entgegenhaltungen :
**EP-A- 0 064 277**
**EP-A- 0 064 278**
**WO-A-82 /011 27**
**DE-A- 2 021 320**
**FR-A- 2 254 302**
**FR-A- 2 327 758**
**FR-A- 2 383 656**
**FR-A- 2 392 661**
**GB-A- 2 025 238**
**GB-A- 2 041 759**
**US-A- 3 919 723**
**US-A- 4 234 972**

(73) Patentinhaber : **ERNST LEITZ WETZLAR GMBH**
**Ernst-Leitz-Strasse 30 Postfach 20 20**
**D-6330 Wetzlar 1 (DE)**

(72) Erfinder : **BRÖMER, Heinz**
**Hundsrück 7**
**D-6330 Wetzlar-Hermannstein (DE)**
Erfinder : **DEUTSCHER, Klaus**
**Lerchenweg 20**
**D-6330 Wetzlar (DE)**
Erfinder : **FRANEK, Henning**
**Mühlenkopfstrasse 5**
**D-6333 Braunfels-Tiefenbach (DE)**
Erfinder : **RECK, Ralf**
**Südring 189**
**D-6500 Mainz-Bretzenheim (DE)**

## Beschreibung

Die Erfindung betrifft eine aus bioaktivem Material bestehende Gehörknöchelchen-Prothese, enthaltend eine Platte, die eine spiegelsymmetrische Form bezüglich einer auf der Plattenebene senkrecht stehenden Symmetrieebene aufweist, einen an der Plattenunterseite derart exzentrisch angesetzten Schaft, daß dessen Ansatzfläche auf der Spur dieser Symmetrieebene, jedoch mindestens um den Halbmesser des Schaftquerschnitts vom Randbereich der Platte entfernt liegt, und eine auf der Plattenoberseite angebrachte Rille, die auf der der Ansatzfläche gegenüberliegenden Hälfte der Platte angeordnet und senkrecht zu der Symmetrieebene ausgerichtet ist.

Die Erfindung betrifft ferner ein Verfahren zur Herstellung dieser Prothese.

Eine derartige Gehörknöchelchen-Prothese wird in der am 1. Oktober 1980 angemeldeten und am 15. April 1982 veröffentlichten Internationalen Patentanmeldung WO-A-82/01127, für welche die Priorität vom 26. September 1980 aus der Voranmeldung DE 3036245.9 in Anspruch genommen ist, beschrieben.

Prothesen dieser Art werden zur vollständigen oder teilweisen Rekonstruktion des im Mittelohrbereich befindlichen Gehörknöchelchen-Traktes verwandt. Insbesondere ist eine ausgezeichnete akustomechanische Kopplung zwischen dem schallaufnehmenden Tympanum und — bei Fehlen eines funktionsfähigen Hammer- und/oder Amboß-Knöchelchens — dem schallweiterleitenden Stapes-Bügelkopf bzw. der Stapes-Fußplatte durch lokalanatomisch optimalen Paßsitz des Implantates realisierbar.

Entscheidende Voraussetzung für diese günstigen otochirurgischen Befunde ist die Verwendung von bioaktivem Prothesenmaterial, insbesondere Bioglaskeramik oder Bioglas.

Bekanntlich weisen derartige bioaktiven Knochenersatzmaterialien eine gewisse Oberflächenlöslichkeit auf, welche vermutlich eine für das Zustandekommen des Knochen/Implantat — Verbundes notwendige Voraussetzung darstellt. Andererseits kann diese spezielle Eigenschaft unter besonders ungünstigen lokalanatomischen Bedingungen, wie sie insbesondere bei der Implantation einer Prothese im Weichgewebelager bzw. bei Kontaktierung einer derartigen Prothese mit Weichgewebe gegeben sind, dazu führen, daß die Langzeitstabilität des Implantates beeinträchtigt wird.

Der Erfindung liegt daher die Aufgabe zugrunde, bei Gehörknöchelchen-Prothesen aus bioaktivem Material diejenigen Oberflächenteilbereiche, die nicht für den eigentlichen Verbund mit dem Knochen (Verwachsungszone : Implantat/knöchernes Hartgewebe) vorgesehen sind, derart auszubilden, daß sie biochemisch vollkommen resistent und bioaktiv sind. Darüber hinaus besteht die Aufgabe darin, ein Verfahren zur nachträglichen Bioinaktivierung von ausgewählten Teilbereichen an sich bioaktiver Gehörknöchelchen-Prothesen anzugeben.

Die Aufgabe wird bei Prothesen der eingangs genannten Art dadurch gelöst, daß in einem oder mehreren Teilbereich(en) der Prothese das bioaktive Material durch unlösliches bioinertes Material ersetzt ist. Sie wird ferner dadurch gelöst, daß — ausgehend von einer ein- oder zweistückigen, aus bioaktivem Material bestehenden Prothese — Teilbereiche dieser Prothese oder Prothesenteile (Platte ; Schaft) entweder unter gezielter Stoffzufuhr einer Oberflächen-Nachbehandlung zur Erzeugung mindestens einer additiv aufgebrachten oder unter gezielter Stoffabfuhr bzw. Stoffaustausch einer chemischen Nachbehandlung zur Erzeugung mindestens einer subtraktiv erhaltenen, dauerhaft festsitzenden, in vivo als biochemische Sperrschicht wirkenden, bioinerten Schutzschicht bzw. Konversions-(Auslaugungs-)Schicht unterworfen werden.

Weitere Ausgestaltungen sind in den anderen Ansprüchen beschrieben.

In den Zeichnungen sind Ausführungsbeispiele der Erfindung schematisch dargestellt, wobei jeweils bei der Darstellung der Seiten- und der Vorderansicht der erfindungsgemäßen Gehörknöchelchen-Prothese von der in der WO-A-82/01127 in der dortigen Fig. 1 und 2 gezeigten Schiefschaftanordnung mit querliegender Rille ausgegangen wird und — soweit möglich — gleiche Bezugsziffern gewählt werden.

Es zeigen :

Figuren 1 und 2 : eine Prothese mit teilweiser Beschichtung (additive Schicht) des Schaftes ;

Figuren 3 und 4 : eine zweistückige Prothese, bestehend aus einem aus bioinertem Material gefertigten Schaft und einer aus bioaktivem Material gefertigten Platte ;

Figuren 5 und 6 : eine weitere Ausführungsform, bei der die additive Schicht den gesamten Schaft sowie die Plattenunterseite mitumfaßt ;

Figuren 7 und 8 : eine weitere Ausführungsform mit inkorporierter Teil-Beschichtung (subtraktive Schicht) des Schaftes.

Die Gehörknöchelchen-Prothese besteht aus einer Platte 1, auf deren Oberseite 8 eine exzentrisch und senkrecht zur Zeichenebene verlaufende Rille 7 angeordnet ist. An der Plattenunterseite 3 ist ein Schaft 2 exzentrisch angeordnet.

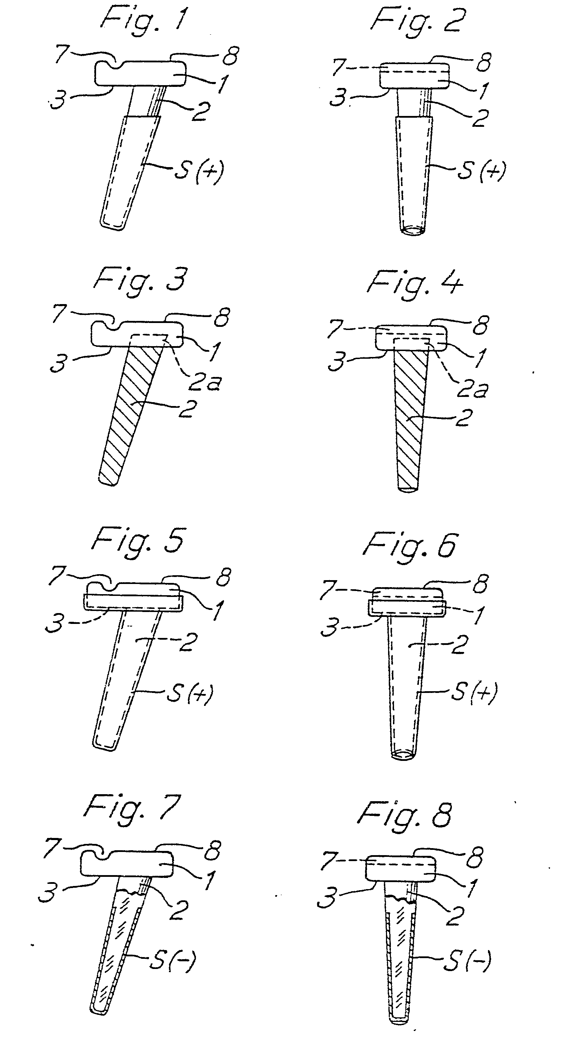

Die Prothese kann einstückig aus bioaktivem Material hergestellt sein, wie es etwa in den Fig. 5 und 6 dargestellt ist. Es ist aber ebenfalls möglich, sie aus mindestens zwei Teilen zusammenzusetzen, wobei entweder beide Teile — also der Schaft 2 und die Platte 1 — aus bioaktivem Material bestehen oder die Platte aus bioaktivem und der Schaft aus bioinaktivem, unlöslichem — also : « bioinertem » — Material besteht. Im Falle der zweistückigen Prothesenform kann der Schaft 2 in einem Blindloch 2a in festem Paßsitz gehaltert sein, vgl. Figuren 3 und 4. Dieser kann beispeilsweise dadurch realisiert werden, daß der in das Blindloch 2a eingreifende Teil des Schaftes

2 mit verengter Toleranz maßgefertigt und unmittelbar vor Einfügen einer lokalen Kältebehandlung unterworfen wird. Andere Einpaßmethoden, wie Nieten, Schrauben, Keilen oder Kleben, sind prinzipiell ebenfalls anwendbar.

In den Fig. 1, 2, 5 und 6 wird die additiv aufgebrachte Bioinert-Schicht S(+) dargestellt. Es ist ein wesentliches Merkmal der vorliegenden Erfindung, nur an denjenigen Oberflächen-Teilbereichen der Prothese bioaktives Material vorzusehen, die in vivo in direktem Kontakt mit knöchernem Gewebe kommen, und an denjenigen Oberflächen-Teilbereichen der Prothese, die in vivo in Kontakt mit Weichgewebe — z. B. Epithel — kommen, gewissermaßen eine biochemische « Korrosions »-Schutzschicht vorzusehen, die eine komplette Transport- und Durchlaß-Barriere für jedweden Stoff-(Ionen)Austausch zwischen den chemischen Bestandteilen des Implantates und den Bestandteilen der physiologisch-biochemischen Körperflüssigkeiten gewährleistet.

Eine Schutzschicht S(−) kann, wie in den Fig. 7 und 8 dargestellt, in vitro auch dadurch erhalten werden, daß bei einer aus bioglaskeramischem Voll-Material oder aus Bioglas-Material bestehenden Gehörknöchelchen-Prothese diejenigen Oberflächen-Teilbereiche, die zwangsweise — also aufgrund anatomischer Gegebenheiten — oder vom Otochirurgen gewollt — also beispeilsweise bei dem Auskleiden bestimmter Implantatbereiche mit Epithelgewebe — mit Weichgewebe in Dauerkontakt kommen, chemisch in der Weise vorbehandelt werden, daß wässrige saure Lösungen und/oder wässrige Salzlösungen in Normalitäten zwischen 0,001 und 0,1 die ursprüngliche bioglaskeramische Oberfläche attackieren, wobei Angriffs-(Auflösungs- bzw. Auslaugungs-) und Austausch-Reaktionen nebeneinander ablaufen mit der Folge, daß es zunächst zu einer Verarmung und schließlich zu einer vollständigen Zerstörung (Umwandlung) einer Phase — insbesondere der kristallinen Komponente(n) — des glaskeramischen Verbundsystems kommt.

Zum chemischen Angriff können aber auch Basen und Puffersysteme verwendet werden, die in Abhängigkeit von ihrem konkreten Chemismus, ihrer Konzentration und ihrem $p_H$-Wert gezielt auf bestimmte Phasenkomponenten des Bioglaskeramik-« Verbundsystems » einwirken und so die gewünschte Eigenschaftsänderung der Implantatoberfläche bewirken.

Die nach dieser kombinierten chemischen Behandlung verbleibende Glaskeramik, die ihrer Apatitanteile beraubt ist, sei mit « Residual-Bioglaskeramik » bezeichnet. Sie ist bezüglich ihrer chemischen Eigenschaften unlöslich, porenfrei und stoppt jeglichen Ionentransport ; sie ist bezüglich ihrer biochemischphysiologischen Wirkung bioinaktiv, also bio-« inert », und bezüglich ihrer mechanischen Eigenschaften abriebresistent und auf dem Prothesenkernmaterial festhaftend.

Im Bedarfsfall kann die so erhaltene Schicht S(−) zusätzlich auf thermischem Wege verdichtet bzw. versiegelt werden. Darüber hinaus ist auch eine Silanisierungsschicht zusätzlich aufbringbar.

Sollte aufgrund eines gegebenen pathologischen Befundes das von der Prothesenplatte 1 wegweisende Prothesenschaft-Ende in direktem Knochenkontakt mit einem Gehörknöchelchen-Relikt (Stapes-Kopf) kommen, wäre die bioinerte Schutzschicht S(+) bzw. S(−) im Kontaktbereich vorher zu entfernen, um an dieser Stelle nach Wunsch des HNO-Chirurgen optimale Verwachsungsverhältnisse zu erreichen. Auf der Plattenoberseite 8 wird vor dem eigentlichen Implantationsvorgang durch Aufbringen von partikulärem, autologem Knochenmehl die Herausbildung einer quasi-kompakten Knochenlamellen-Schicht induziert, die als Kontakt-Zwischenzone zwischen der Gehörknöchelchen-Prothesenplattenoberseite und dem Tympanum fungiert.

**Patentansprüche**

1. Aus bioaktivem Material bestehende Gehörknöchelchen-Prothese, enthaltend eine Platte (1), die eine spiegelsymmetrische Form bezüglich einer auf der Plattenebene senkrecht stehenden Symmetrieebene aufweist, einen an der Plattenunterseite (3) derart exzentrisch angesetzten Schaft (2), daß dessen Ansatzfläche auf der Spur dieser Symmetrieebene, jedoch mindestens um den Halbmesser des Schaftquerschnitts vom Randbereich der Platte entfernt liegt, und eine auf der Plattenoberseite (8) angebrachte Rille (7), die auf der der Ansatzfläche gegenüberliegenden Hälfte der Platte angeordnet und senkrecht zu der Symmetrieebene ausgerichtet ist, dadurch gekennzeichnet, daß einem oder mehreren Teilbereich(en) der Prothese das bioaktive Material durch unlösliches bioinertes Material ersetzt ist.

2. Gehörknöchelchen-Prothese nach Anspruch 1, dadurch gekennzeichnet, daß der Teilbereich ein Oberflächen-Teilbereich ist.

3. Gehörknöchelchen-Prothese nach einem der vorhergehenden Ansprüche, dadurch gekennzeichnet, daß sie partiell eine Beschichtung aus unlöslichem bioinerten Material aufweist.

4. Gehörknöchelchen-Prothese nach Anspruch 3, dadurch gekennzeichnet, daß die Beschichtung aus mindestens einer additiv aufgebrachten Schutzschicht (S(+)) mit einer Dicke zwischen 0,25 und 10 μm besteht.

5. Gehörknöchelchen-Prothese nach Anspruch 3, dadurch gekennzeichnet, daß die Beschichtung aus mindestens einer subtraktiv erzeugten Konversionsschicht (S(−)) mit einer Dicke zwischen 0,25 und 5 μm besteht.

6. Gehörknöchelchen-Prothese nach den Ansprüchen 3 bis 5, dadurch gekennzeichnet, daß die Beschichtung aus mindestens einer prothesenkernseitig erzeugten Konversionsschicht und mindestens einer auf dieser aufliegenden, additiv aufgebrachten Schutzschicht besteht.

7. Gehörknöchelchen-Prothese nach mindestens einem der vorhergehenden Ansprüche, dadurch gekennzeichnet, daß der Prothesenschaft zumindest in seinem Mantelbereich aus unlöslichem bioinerten Material besteht.

8. Gehörknöchelchen-Prothese nach mindestens einem der vorhergehenden Ansprüche, dadurch gekennzeichnet, daß lediglich die Platte aus bioaktivem Material besteht.

9. Gehörknöchelchen-Prothese nach mindestens einem der vorhergehenden Ansprüche, dadurch gekennzeichnet, daß die Platte aus bioaktivem und der Schaft aus bioinertem Vollmaterial bestehen.

10. Gehörknöchelchen-Prothese nach mindestens einem der vorhergehenden Ansprüche, dadurch gekennzeichnet, daß die Platte aus bioaktivem Material und der Schaft zumindest in seinem Mantelbereich aus unlöslichem bioinerten Material bestehen.

11. Gehörknöchelchen-Prothese nach mindestens einem der Ansprüche 1 bis 4, 6, 7, 9 und 10, dadurch gekennzeichnet, daß das bioinerte Material bzw. die bioinerte Schicht aus mindestens einer der folgenden Substanzen besteht :
    a) Metalle, wie Gold, Platin, Titan, sowie Metallegierungen ;
    b) Kohlenstoff in geeigneten Modifikationen, wie pyrolytischer Kohlenstoff (Graphit) ; Kohlenstoffverbindungen, wie Siliziumkarbid (SiC), Titankarbid (TiC), Borkarbid ($B_4C$) ;
    c) Sonderkeramische Werkstoffe, wie hexagonales Bornitrid (BN), Titannitrid (TiN), Siliciumnitrid ($Si_3N_4$) ;
    d) teilkristalline anorganische Verbundsysteme, wie Emails ;
    e) anorganische Einkomponenten- (z. B. Kieselglas) oder Mehrkomponenten-Gläser ;
    f) Oxide, wie Titandioxid ($TiO_2$), Zirkondioxid ($ZrO_2$) und Aluminiumoxid ($Al_2O_3$).

12. Gehörknöchelchen-Prothese nach mindestens einem der Ansprüche 1 bis 3, 5 bis 7 und 10, dadurch gekennzeichnet, daß das bioinerte Material bzw. die bioinerte Schicht aus einer(m) apatitfreien Residual-Bioglaskeramik bzw. -Biokeramik bzw. -Bioglas besteht, welche(s) gegebenenfalls zusätzlich eine Silanschicht aufweist.

13. Verfahren zur Herstellung einer Gehörknöchelchen-Prothese nach mindestens einem der vorhergehenden Ansprüche, dadurch gekennzeichnet, daß — ausgehend von einer ein- oder zweistückigen, aus bioaktivem Material bestehenden Prothese — Teilbereiche dieser Prothese oder Prothesenteile (Platte ; Schaft) unter gezielter Stoffzufuhr einer Oberflächen-Nachbehandlung zur Erzeugung mindestens einer additiv aufgebrachten, dauerhaft festsitzenden, in vivo als biochemische Sperrschicht wirkenden, bioinerten Schutzschicht (S(+)) unterworfen werden.

14. Verfahren zur Herstellung einer Gehörknöchelchen-Prothese nach mindestens einem der Ansprüche 1 bis 12, dadurch gekennzeichnet, daß — ausgehend von einer ein- oder zweistückigen, aus bioaktivem Material bestehenden Prothese — Teilbereiche dieser Prothese oder Prothesenteile (Platte ; Schaft) unter gezielter Stoffabfuhr bzw. Stoffaustausch einer chemischen Nachbehandlung zur Erzeugung mindestens einer subtraktiv erhaltenen, permanent festhaftenden, in vivo als biochemische Sperrschicht wirkenden, bioinerten Konversions-(Auslaugungs-)Schicht (S(−)) unterworfen werden.

15. Verfahren nach Anspruch 13, dadurch gekennzeichnet, daß die Schutzschicht (S(+)) durch mindestens einen der folgenden Schritte vorzugsweise aufgebracht wird :
    a) Galvanisieren oder Bedampfen ;
    b) Aufsputtern ;
    c) Abscheiden aus organischen Lösungen oder Aufdampfen im Vakuum ;
    d) Tauchen, Sprühen oder Aufstreuen mit nachfolgender thermischer Behandlung ;
    e) Eintauchen in Wasserglas mit anschließendem Aufheizen auf ca. 400 °C bzw. Tauchglasieren aus ein- oder mehrkomponentigen Schmelzgemengen ;
    f) Simultan-Aufdampfen oder Aufdampfen der Metalle mit nachfolgender Oxidierungsbehandlung.

16. Verfahren nach Anspruch 14, dadurch gekennzeichnet, daß die zu schützenden Prothesenteile mit wässrigen, sauren Lösungen oder mit wässrigen Salzlösungen mit Normalitäten zwischen 0,001 und 0,1 zwischen 5 Minuten und 3 Stunden bei Temperaturen zwischen 20 bis 100 °C behandelt werden.

17. Verfahren nach Anspruch 16, dadurch gekennzeichnet, daß als wässrige, saure Lösung 0,1-0,001 normale Salzsäure (HCl) verwendet wird.

18. Verfahren nach Anspruch 16, dadurch gekennzeichnet, daß als wässrige Salzlösung 0,001 bis 0,25 normale Standard-Azetat-Puffer-Lösung verwendet wird.

19. Verfahren nach Anspruch 14, dadurch gekennzeichnet, daß die beschichteten Teile der Prothese anschließend einer thermischen Versiegelungs- und/oder Silanisierungsbehandlung unterworfen werden.

**Claims**

1. Prosthesis, for auditory ossicles, consisting of bio-active material and containing a plate (1), which displays a specularly symmetrical shape with respect to a plane of symmetry standing perpendicularly on the plane of the plate, a shank (2) placed eccentrically against the underside (3) of the plate in such a manner that its connecting area lies on the track of this plane of symmetry, however spaced from the rim region of the plate by at least half the diameter of the shank cross-section, and a groove (7), which is applied on the upper side (8) of the plate, arranged on the plate half lying opposite the connecting area and aligned perpendicularly to the plane of symmetry, characterised thereby, that the bio-active material in one or other partial region or other partial regions is replaced by insoluble bio-inert material.

2. Prosthesis, for auditory ossicles, according to claim 1, characterised thereby, that the partial region is a partial surface region.

3. Prosthesis, for auditory ossicles, according to one of the preceding claims, characterised thereby, that it in part displays a coating of insoluble bio-inert material.

4. Prosthesis, for auditory ossicles, according to claim 3, characterised thereby, that the coating consists of at least one additively applied protective layer (S(+)) of a thickness between 0.25 and 10 micrometres.

5. Prosthesis, for auditory ossicles, according to claim 3, characterised thereby, that the coating consists of at least one subtractively generated conversion layer (S(−)) of a thickness between 0.25 and 5 micrometres.

6. Prosthesis, for auditory ossicles, according to the claims 3 to 5, characterised thereby, that the coating consists of at least one conversion layer produced at the side of the core of the prosthesis and at least one additively applied protective layer lying on the conversion layer.

7. Prosthesis, for auditory ossicles, according to at least one of the preceding claims, characterised thereby, that the prosthesis shank at least in its shell region consists of insoluble bio-inert material.

8. Prosthesis, for auditory ossicles, according to at least one of the preceding claims, characterised thereby, that merely the plate consists of bio-active material.

9. Prosthesis, for auditory ossicles, according to at least one of the preceding claims, characterised thereby, that the plate consists of bio-active and the shank of bio-inert solid material.

10. Prosthesis, for auditory ossicles, according to at least one of the preceding claims, characterised thereby, that the plate consists of bio-active material and the shank at least in its shell region consists of insoluble bio-inert material.

11. Prosthesis, for auditory ossicles, according to at least one of the claims 1 to 4, 6, 7, 9 and 10, characterised thereby, that the bio-inert material or the bio-inert layer consists of at least one of the following substances :

a) metals such as gold, platinum, titanium as well as metal alloys,

b) carbon in suitable modifications, such as pyrolytic carbon (graphite), carbon compounds such as silicon carbide (SiC), titanium carbide (TiC) and boron carbide (B$_4$C),

c) special ceramic materials such as hexagonal boron nitride (BN), titanium nitride (TiN) and silicon nitride (Si$_3$N$_4$),

d) partially crystalline inorganic compound systems such as enamels,

e) inorganic single-component glasses (for example flint glass) or multi-component glasses, and

f) oxides such as titanium dioxide (TiO$_2$),

zirconium dioxide (ZrO$_2$) and aluminium oxide (Al$_2$O$_3$).

12. Prosthesis, for auditory ossicles, according to at least one of the claims 1 to 3, 5 to 7 and 10, characterised thereby, that the bio-inert material or the bio-inert layer (S(−)) consists of a residual bioglass ceramic material or a residual bioceramic material or a bioglass, which is apatite-free and in a given case additionally displays a silane layer.

13. Process for the manufacture of a prosthesis, for auditory ossicles, according to at least one of the preceding claims, characterised thereby, that starting out from a one-piece or two-piece prosthesis consisting of bio-active material, partial regions of this prosthesis or prosthesis parts (plate ; shank) are subjected under pinpointed substance supply to a surface-finishing treatment for the generation of at least one additively applied, permanently adhering, inorganic bio-inert protective layer (S(+)), which in vivo acts as biochemical barrier layer.

14. Process for the manufacture of a prosthesis, for auditory ossicles, according to at least one of the claims 11 to 12, characterised thereby, that starting out from a one-piece or two-piece prosthesis consisting of bio-active material, partial regions of this prosthesis or prosthesis parts (plate ; shank) are subjected under pinpointed substance removal or substance exchange to a chemical finishing treatment for the generation of at least one subtractively obtained, permanently adhering bio-inert conversion (leaching) layer (S(−)), which in vivo acts as biochemical barrier layer.

15. Process according to claim 13, characterised thereby, that the protective layer (S(+)) is preferably applied through at least one of the following steps :

a) galvanising or vapour-depositing,

b) sputtering,

c) precipitating out of organic solutions or vapourising under vacuum,

d) dipping, spraying or scattering-on with subsequent thermal treatment,

e) immersion in water glass with subsequent heating to about 400 °C or immersion-glazing from single-component or multi-component smelt batches and

f) simultaneous evaporation or evaporation of the metals with subsequent oxidising treatment.

16. Process according to claim 14, characterised thereby, that the prosthesis parts to be protected are treated with aqueous acid solutions or with aqueous salt solutions of molecular concentrations between 0.001 and 0.1 for between 5 minutes and 3 hours at temperatures between 20 to 100 °C.

17. Process according to claim 16, characterised thereby, that hydrochloric acid (HCl) of a molecular concentration between 0.1 and 0.001 is used as aqueous acid solution.

18. Process according to claim 16, characterised thereby, that standard acetate buffer solution of a molecular concentration between 0.001 and 0.25 is used as aqueous salt solution.

19. Process according to claim 14, characterised thereby, that the coated parts of the prosthesis are subsequently subjected to a thermal sealing treatment and/or silanising treatment.

## Revendications

1. Prothèse d'osselets d'oreille consistant en matière bioactive et contenant une plaque (1) qui présente une forme spéculairement symétrique relativement à un plan de symétrie placé perpendiculairement au plan de la plaque, une tige (2) rattachée excentriquement de telle façon à la face inférieure de plaque (3) que sa surface de raccordement est située sur la trace de ce plan de symétrie mais éloignée de la zone marginale de la plaque d'au moins le rayon de la section transversale de tige, et une rigole (7) aménagée sur la face supérieure de plaque (8) et qui est disposée sur la moitié de la plaque opposée à la surface de rattachement et est orientée perpendiculairement au plan de symétrie, caractérisée en ce que, dans une ou plusieurs régions partielles de la prothèse, la matière bioactive est remplacée par de la matière insoluble bio-inerte.

2. Prothèse d'osselets d'oreille selon la revendication 1, caractérisée en ce que la région partielle est une région partielle de surface.

3. Prothèse d'osselets d'oreille selon l'une des revendications précédentes, caractérisée en ce qu'elle présente partiellement un revêtement en couche, d'une matière insoluble bio-inerte.

4. Prothèse d'osselets d'oreille selon la revendication 3, caractérisée en ce que le revêtement consiste en au moins une couche de protection (S(+)) appliquée additivement avec une épaisseur entre 0,25 et 10 µm.

5. Prothèse d'osselets d'oreille selon la revendication 3, caractérisée en ce que le revêtement consiste en au moins une couche de conversion (S(−)) produite soustractivement avec une épaisseur entre 0,25 et 5 µm.

6. Prothèse d'osselets d'oreille selon les revendications 3 à 5, caractérisée en ce que le revêtement se compose d'au moins une couche de conversion produite du côté du noyau de la prothèse et d'au moins une couche de protection appliquée sur celle-ci et rapportée additivement.

7. Prothèse d'osselets d'oreille selon au moins l'une des revendications précédentes, caractérisée en ce que la tige de prothèse consiste, au moins dans sa zone de paroi latérale, en une matière insoluble bio-inerte.

8. Prothèse d'osselets d'oreille selon au moins l'une des revendications précédentes, caractérisée en ce que seule la plaque est constituée en matière bioactive.

9. Prothèse d'osselets d'oreille selon au moins l'une des revendications précédentes, caractérisée en ce que la plaque consiste en matière bioactive et la tige en matière pleine bio-inerte.

10. Prothèse d'osselets d'oreille selon au moins l'une des revendications précédentes, caractérisée en ce que la plaque consiste en matière bioactive et la tige, au moins dans sa zone de paroi latérale, en matière insoluble bio-inerte.

11. Prothèse d'osselets d'oreille selon au moins l'une des revendications 1 à 4, 6, 7, 9 et 10, caractérisée en ce que la matière bio-inerte ou la couche bio-inerte consiste en au moins l'une des substances suivantes :

a) des métaux tels que l'or, le platine, le titane ainsi que des alliages métalliques ;

b) du carbone en des variétés appropriées telles que du carbone pyrolytique (graphite) ; des combinaisons de carbone, telles que le carbure de silicium (SiC), le carbure de titane (TiC), le carbure de bore ($B_4C$) ;

c) des matières céramiques spéciales telles que le nitrure de bore (BN), le nitrure de titane (TiN), le nitrure de silicium ($Si_3N_4$) ;

d) des systèmes composites inorganiques partiellement cristallins tels que des émaux ;

e) des verres inorganiques à un composant (par exemple quartz fondu) ou à plusieurs composants ;

f) des oxydes tels que le dioxyde de titane ($TiO_2$), le dioxyde de zirconium ($ZrO_2$) et l'oxyde d'aluminium ($Al_2O_3$).

12. Prothèse d'osselets d'oreille selon au moins l'une des revendications 1 à 3, 5 à 7 et 10, caractérisée en ce que la matière bio-inerte se compose d'un biovitrocérame ou d'une biocéramique ou d'un bioverre résiduel exempt d'apatite, lequel éventuellement comporte additionnellement une couche de silane.

13. Procédé de fabrication d'une prothèse d'osselets d'oreille selon au moins l'une des revendications précédentes, caractérisé en ce qu'en partant d'une prothèse constituée en une ou en deux pièces en matière bioactive, des régions partielles de cette prothèse ou des éléments de prothèse (plaque ; tige) sont soumis à un traitement de surface subséquent sous apport judicieux de substance pour la production d'au moins une couche de protection bio-inerte (S(+)) rapportée additivement, fixée de façon permanente et agissant in vivo comme couche de barrage biochimique.

14. Procédé de fabrication d'une prothèse d'osselets d'oreille selon au moins l'une des revendications 1 à 12, caractérisé en ce qu'en partant d'une prothèse constituée en une ou en deux pièces en matière bioactive, des régions partielles de cette prothèse ou des éléments de prothèse (plaque ; tige) sont soumis à un post-traitement chimique, sous enlèvement de substance ou échange de substance judicieux, pour la production d'au moins une couche de conversion (de macération) bio-inerte (S(−)) obtenue soustractivement, fixement adhérente de façon permanente et agissant in vivo comme couche de barrage biochimique.

15. Procédé selon la revendication 13, caractérisé en ce que la couche de protection (S(+)) est de préférence appliquée par au moins l'une des opérations suivantes :

a) galvanoplastie ou dépôt par vaporisation ;

b) dépôt par pulvérisation cathodique ;

c) séparation de solutions organiques ou métallisation sous vide ;

d) trempage, pulvérisation ou saupoudrage avec traitement thermique consécutif ;

e) immersion dans du verre soluble avec chauffage consécutif jusqu'à environ 400 °C ou vitrification par immersion à partir de mélanges fondus à un ou plusieurs composants ;

f) métallisation simultanée ou dépôt de métaux par évaporation sous vide avec traitement d'oxydation consécutif.

16. Procédé selon la revendication 14, caractérisé en ce que les parties de prothèse à protéger sont traitées avec des solutions acides aqueuses ou avec des solutions salines aqueuses ayant des normalités entre 0,001 et 0,1 pendant une durée comprise entre 5 minutes et 3 heures à des températures de 20 à 100 °C.

17. Procédé selon la revendication 16, caractérisé en ce que comme solution acide aqueuse est utilisée une solution saline (HCl) 0,1-0,001 fois normale.

18. Procédé selon la revendication 16, caractérisé en ce que comme solution saline aqueuse est employée une solution tampon d'acétate standard 0,001 à 0,25 fois normale.

19. Procédé selon la revendication 14, caractérisé en ce que les parties revêtues de la prothèse sont soumises subséquemment à un traitement thermique de scellement et/ou de silanisation.

Fig. 1

Fig. 2

Fig. 3

Fig. 4

Fig. 5

Fig. 6

Fig. 7

Fig. 8